# FASCICULE DE BREVET EUROPEEN

(11) **EP 0 416 004 B1**
(45) Date de publication et mention de la délivrance du brevet: **29.12.1993**
(21) Numéro de dépôt: 89906376.2
(22) Date de dépôt: 26.05.1989
(51) Int. Cl.: A61B 17/44

(54) **Appareil d'extraction de fetus par les voies genitales naturelles maternelles**
Extraktionsgerät für die Geburtshilfe
Apparatus for extracting a fetus through the female genital tracks

(30) Priorité: 26.05.1988 FR 8807004
(43) Date de publication de la demande: 13.03.1991
(73) Titulaire: BERNON, Gilles, F-77200 Torcy (FR)
(72) Inventeur: BERNON, Gilles, F-77200 Torcy (FR)
(74) Mandataire: Ores, Irène
(86) Numéro de dépôt international: FR8900252
(87) Numéro de publication internationale: WO8911253

(56) Documents cités:
- DE-A- 2 233 840
- DE-A- 2 925 386
- US-A- 3 794 044

## Description

La présente invention est relative à un instrument d'extraction de foetus par la voie génitale naturelle,c'est à dire par la voie vaginale.

Il existe actuellement trois types d'instruments destinés à saisir la tête d'un foetus pour l'extraire des voies génitales naturelles de la mère, dans le but d'abréger la durée de l'accouchement en cas de souffrance foetale ou d'arrêt de la progression de la tête foetale. Ces instruments sont le forceps, l'extracteur a dépression,ou ventouse,et les spatules.

Comme on le sait, le forceps est une pince à deux branches qui remonterait à l'époque romaine mais dont les formes modernes datent du 16ème siècle. Il existe des forceps à branches croisées et des forceps à branches parallèles. Les forceps à branches croisées, articulées entre elles et dont les "mors" ou "cuillers" de la pince présentent une courbure céphalique s'adaptant à la tête foetale et une courbure pelvienne s'adaptant au plancher pelvien maternel, comportent des moyens de traction des cuillers dont les mieux adaptés sont inclus dans le forceps de TARNIER.

Le forceps à branches parallèles mis au point par DEMELIN élimine les problèmes d'articulation posés par les forceps à branches croisées. Dans ce second type de forceps, les branches non croisées, articulées par une vis, au niveau des manches qui prolongent les cuillers, que l'on peut serrer à volonté, protégeant ainsi la tête foetale, ont été remplacés par des forceps à branches parallèles, solidarisées par une branche transversale et sur lesquelles on peut fixer des lacs.

Il faut noter qu'il avait été proposé par CHASSAGNY, en 1860, de fixer des lacs à des branches portées par les cuillers de forceps à branches croisées, et destinées à permettre l'application de forces de traction aux cuillers à proximité de la tête foetale; cependant ces lacs ne pouvaient respecter la courbure pelvienne, en sorte que ce forceps fut rapidement abandonné au profit du forceps de TARNIER.

C'est en 1950 qu'ont été proposées les spatules de THIERRY constituées de deux cuillers tout à fait indépendantes, qui ne constituent donc pas une pince. Ces deux spatules symétriques comportent une cuiller pleine à courbure faciale et à courbure pelvienne,et un manche rectiligne présentant à son extrêmité libre des encoches pour recevoir les doigts de l'accoucheur; elles ont pour but de faire reproduire à la tête foetale des mouvements proches des mouvements physiologiques, mais en respectant sa mobilité.

Les ventouses obstétricales d'extraction de foetus se composent de trois éléments principaux : un système de préhension, un dispositif de traction et un générateur de vide.

Le système de préhension est constitué par une cupule, ou ventouse métallique, de diamètre approprié pour présenter une adhérence suffisante: Le dispositif de traction se compose d'une plaque métallique maintenue à une légère distance de la ventouse et au centre de laquelle est fixée une chaînette métallique qui chemine à l'intérieur d'un tube en caoutchouc souple lui-même raccordé à l'orifice externe de la ventouse et à l'extrêmité opposée duquel s'adapte une poignée métallique cruciforme dont l'une des branches creuse permet l'introduction d'une tige embrochant la chaîne de traction. Le générateur de vide se compose d'une pompe à vide pourvue d'un manomètre et fonctionnant à la main.

Pour éviter les éventuels effets traumatiques des instruments métalliques, des systèmes flexibles tels que rubans, frondes ou filets ont été proposés pour l'extraction foetale, en particulier au Japon au 18ème siècle; parmi ceux-ci : un collet de baleine pliable destiné à être passé autour de l'extrêmité du foetus et relié à son extrêmité opposée à une poignée en bois qui servait à la traction; un morceau de soie associé à un ruban de soie pour la traction.

L'on connait également l'extracteur obstétrical du Dr John EVANS ( CHICAGO, milieu du 19ème siècle) formé de deux branches d'acier parallèles, avec une courbe pour contourner le fond du sacrum et un joint permettant une mobilité relative près de l'extrêmité du segment courbe, tandis que les barres parallèles étaient maintenues ensemble par un fermoir plat, sur les poignées, pendant l'introduction des extrêmités courbes dans le bassin ; un filet de ruban de soie destiné à enserrer la tête foetale était fixé aux branches d'extrêmité courbes. Après introduction, le fermoir était enlevé, les branches étaient séparées et l'une d'elles était passée du côté opposé du bassin, de façon à encercler la tête foetale dans le filet. A l'aide des longs rubans du filet, qui pendaient du vagin, l'accoucheur exerçait une traction alternativement sur chacune de leurs extrêmités attachées et sur les branches d'acier pour entraîner la descente de la tête foetale. L'efficacité de ce type d'extracteur à branches parallèles, dépourvu de branche transversale ou de vis d'articulation, n'a pas été démontrée, en sorte qu'il ne s'est pas imposé dans la pratique.

Il a également été proposé, par la Demande de Brevet allemand HEEGE N° 2 233 840, un appareil d'obstétrique qui comporte un tuyau réticulé dont la partie antérieure est pourvue d'une bordure dilatable, ce tuyau étant disposé dans une douille de préhension et pouvant être élargi au moins jusqu'à la grosseur d'une tête de nouveau-né à l'aide de tiges de guidage flexibles qui sont montées déplaçables dans ladite douille et s'accrochent à la bordure susdite.

Les instruments d'extraction de la tête foetale, en usage actuellement, présentent un certain nombre d'inconvénients : ils doivent être utilisés par des accoucheurs expérimentés dans leur maniement pour éviter toutes conséquences lésionnelles chez l'enfant ou/et chez la mère, conséquences qu'ils ne sont cependant pas toujours en mesure d'éviter, en raison de la structure métallique rigide des instruments utilisés et de leur principe de fonctionnement.

La présente invention s'est en conséquence donné pour but de pourvoir à un appareil d'extraction de foetus, notamment humains, par les voies génitales maternelles naturelles, qui répond mieux aux nécessités de la pratique que les instruments proposés dans l'Art antérieur, en ce qu'il assure une meilleure préhension de la tête foetale sans risques traumatiques tant pour la mère que pour l'enfant, en ce qu'il évite toute friction en isolant la tête du foetus dans son mouvement de descente, des parties molles du bassin et en ce que sa conception est telle qu'il peut être réalisé à un prix de revient suffisamment bas pour autoriser son utilisation à "usage unique", évitant ainsi les opérations de stérilisation auxquelles doivent habituellement être soumis ces sortes d'instruments.

La présente invention a pour objet un appareil d'extraction de foetus par les voies génitales maternelles naturelles, comprenant une carcasse constituée par une pluralité de tiges flexibles disposées sensiblement en forme de corolle, solidaires, à l'une de leurs extrémités, d'une structure réticulée en matériau souple, ladite structure s'étendant à l'intérieur de la corolle susdite, et une gaine tubulaire creuse, caractérisé en ce que lesdites tiges sont solidaires à leurs extrémités opposées de la gaine tubulaire, et que ladite structure réticulée en matériau souple se compose de deux réseaux imbriqués l'un dans l'autre, dont l'un est un réseau à mailles bloquées et dont l'autre est un réseau à mailles libres, les tresses qui composent le réseau à mailles libres comportant à leur extrémité qui s'étend librement à l'intérieur de la corolle susdite, des brins de traction fixés à ladite extrémité et qui se terminent à leurs extrémités opposées, en un noyau d' ancrage apte à être reçu dans un organe de traction approprié.

Selon un mode de réalisation avantageux de l'appareil conforme à la présente invention, les brins de traction sont guidés dans la gaine tubulaire susdite.

Selon un autre mode de réalisation avantageux de l'appareil conforme à la présente invention, l'organe de traction comprend une cage destinée à recevoir le noyau d'ancrage susdit, sur une partie de laquelle est monté un manchon mobile et dont l'extrémité opposée à celle sur laquelle est monté le manchon porte une poignée de traction.

Selon une disposition avantageuse de ce mode de réalisation, le manchon est monté mobile sur la partie d'extrêmité de la cage d'ancrage orientée vers la gaine tubulaire creuse de l'appareil d'extraction.

Selon une modalité avantageuse de cette disposition, ladite partie d'extrêmité est pourvue d'un filetage correspondant au filetage intérieur dont est également pourvu le manchon susdit.

Selon une autre disposition de réalisation avantageuse de l'organe de traction susdit, le manchon coopère avec une butée solidaire de la gaine tubulaire susdite.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels :

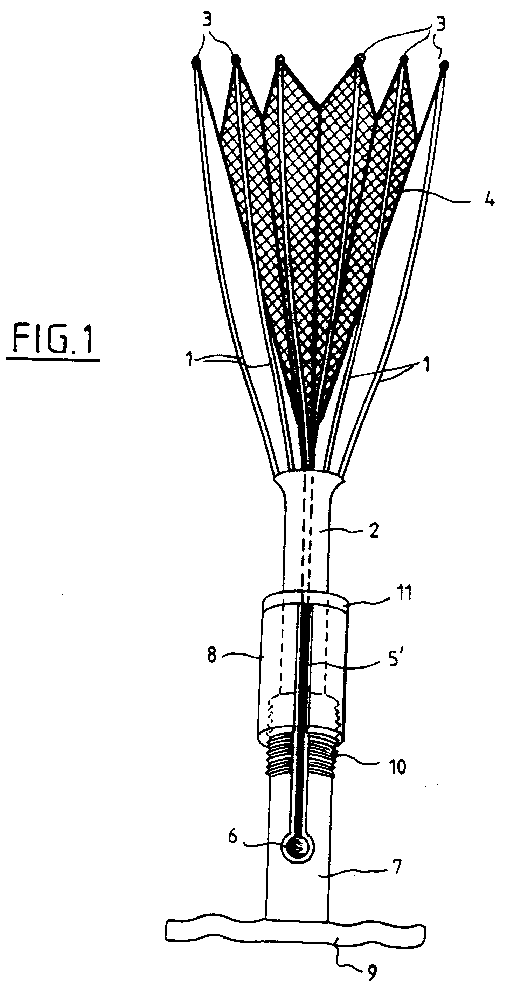

La figure 1 est une vue en élévation latérale d'un appareil d'extraction de la tête foetale, conforme à la présente invention, et

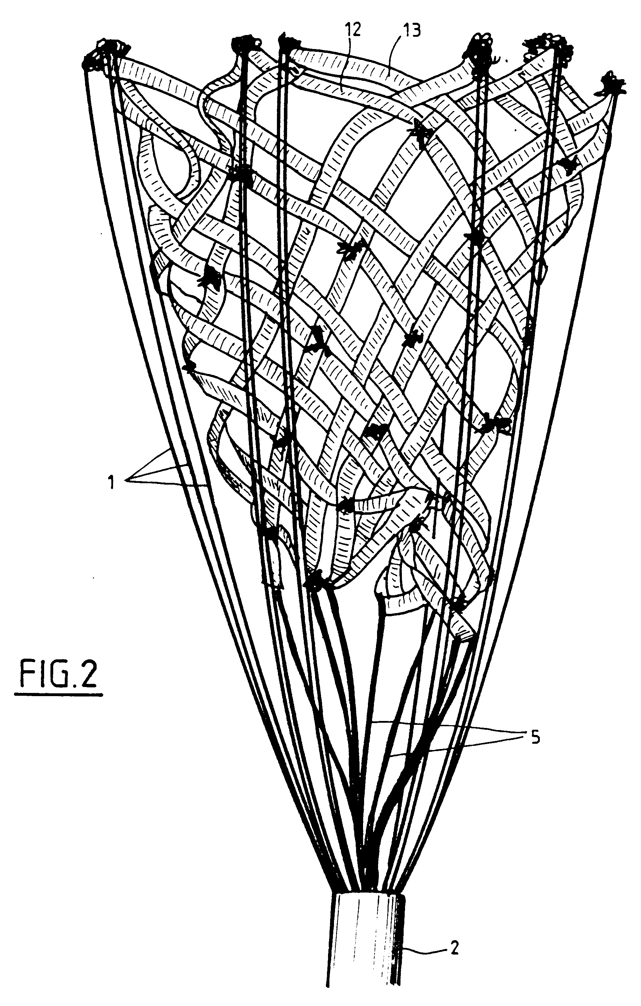

La figure 2 est une vue à plus grande échelle de la structure réticulée souple et de la corolle semi-rigide à laquelle elle est fixée à l'une de ses extrémités.

Il doit être bien entendu, toutefois, que ces dessins et les parties descriptives correspondantes, sont donnés uniquement à titre d'illustration de l'objet de l'invention, dont ils ne constituent en aucune manière une limitation.

L'appareil pour expulsion foetale par les voies génitales maternelles naturelles, conforme à la présente invention, comprend une pluralité de tiges 1 réalisées en un matériau approprié leur conférant une certaine résilience, métal, matière plastique, etc.... Ces tiges 1 sont fixées à l'une de leur extrêmités à une gaine tubulaire creuse 2, tandis qu'à leurs extrêmités libres 3 est fixée une structure réticulée 4 qui s'étend à l'intérieur de la structure sensiblement en forme de corolle déterminée par la réunion des tiges 1 solidarisées à leurs extrémités inférieures à ladite gaine 2. Les dimensions de la structure réticulée 4 sont suffisantes pour lui permettre d' enserrer la tête du foetus lors des opérations d'extraction du foetus.

Des brins de traction 5 sont fixés à la partie inférieure de la structure réticulée 4 et sont guidés dans la gaine 2. C'est la traction de ces brins 5 qui permet le fonctionnement de l'appareil conforme à l'invention et l'expulsion du foetus.

Ces brins 5 sont avantageusement réunis en un cordon 5' qui se termine par un noyau d'ancrage 6 lequel est propre à être reçu dans la cage d'ancrage 7 d'un organe de traction amovible. Ledit organe de traction se compose outre de ladite cage d'ancrage 7, d'un manchon 8 et d'une poignée de traction 9. Le manchon 8 est monté mobile sur la partie d'extrêmité 10 de la cage d'ancrage 7 orientée en direction de la gaine 2 susdite ; la mobilité du manchon 8 lui est conférée en pourvoyant la partie d'extrémité 10 de la cage d'ancrage 7 d'un filetage qui coopère avec un filetage intérieur correspondant dont est pourvu le manchon 8. La poignée de traction 9 est fixée à l'extrêmité de la cage d'ancrage 7 opposée à celle qui porte le filetage, 10. Le mouvement du manchon est limité par la disposition d'un collet de butée 11 fixé sur la gaine 2.

L'organe de traction est facile à associer à l'appareil d'expulsion proprement dit qui se compose de la corolle 1, de la structure réticulée 4 avec les brins de traction 5 et de la gaine-poignée 2.

La structure réticulée, réalisée en matériau souple, se compose de deux réseaux étroitement imbriqués l'un dans l'autre, dont l'un est à mailles bloquées 12, et dont l'autre est formé d'un réseau à mailles libres 13. Les tresses qui forment le réseau 12 et le réseau 13 sont fixées ensemble à chacune des extrêmités libres des tiges 1 qui forment la corolle susdite, et la structure réticulée 4, formée de ces deux réseaux 12 et 13 conjoints, s'étend à l'intérieur de ladite corolle. Alors que les tresses des deux réseaux sont fixées aux extrêmités libres des tiges 1 de la corolle, seules les tresses du réseau à mailles libres 13 sont rendues solidaires, de façon inamovible, des brins de traction 5.

Le matériau souple dont est constituée la structure réticulée 4 est avantageusement, mais non limitativement, un matériau textile approprié ou un matériau de synthèse ou encore une matière souple appropriée.

Les tiges 1 sont avantageusement , mais non limitativement, formées de fils métalliques de préférence semi-rigides et affectés d'une certaine résilience, ou de fils de matière plastique répondant aux mêmes critères. Il est avantageux de revêtir lesdites tiges 1 d'un matériau facilitant le glissement de l'appareil à l'intérieur des voies génitales maternelles, tel que "TEFLON" en particulier.

Le fonctionnement de l'appareil d'extraction foetale conforme à la présente invention est le suivant :
lorsque les conditions de la phase d'expulsion sont réunies et qu'il est souhaité d'abréger la souffrance foetale et/ou les efforts d'expulsion ou de pallier l'arrêt ou l'absence des efforts d'expulsion, on recourt a l'assistance de l'appareil d'expulsion foetale conforme à la présente invention, en procédant comme suit :
la partie "applicateur" de l'appareil, qui comprend la corolle à laquelle est fixée la structure réticulée 4 manoeuvrée par les brins de traction 5 et qui est elle-même fixée à la gaine 2 par les extrêmités inférieures des tiges 1 qui la composent, est introduite par les voies génitales maternelles jusqu'au contact de la tête foetale en exerçant une légère traction sur les brins de traction 5 de préférence réunis en cordon 5', afin de réduire le diamètre de l'applicateur. On relâche alors la traction exercée sur le cordon 5' et l'on engage les tiges 1 autour de la tête foetale. L'organe de traction (qui se compose du manchon 8, de la cage d'ancrage 7 et de la poignée de traction 9) est alors assemblé à la gaine-poignée 2 de l'applicateur, en introduisant simultanément le cordon de traction 5' et le noyau d'ancrage 6 dans la cage d'ancrage 7, puis on visse le manchon 8 sur le filetage 10 de la cage d'ancrage 7 et on l'amène au contact de la collerette de butée 11 montée sur la gaine-poignée 2 de l'applicateur.

La force exercée par le manchon 8 sur la butée 11, provoque la courbure convexe des tiges 1 qui forment la corolle susdite, lesquelles tiges exercent une légère pesée sur les parties molles du bassin,suffisante pour les écarter de la tête du foetus et faciliter sa descente en en écartant les obstacles ; simultanément à l'obtention de ladite courbure convexe des tiges 1, du fait de sa solidarité avec les extrêmités libres des tiges 1 de la corolle, la structure réticulée se resserre autour de la tête foetale autour de laquelle elle forme un filet de préhension atraumatique. L'expulsion est alors réalisée en agissant sur la poignée 9 de traction.

On obtient ainsi une expulsion sans risques traumatiques tant pour la mère que pour l'enfant.

En effet, la convexion réglable des tiges 1, dont les extrêmités libres peuvent avantageusement être spatulées, ouvre le passage de la tête du foetus dans sa descente, en l'isolant des parties molles du bassin, évitant ainsi toute friction ; la matière souple utilisée pour réaliser la structure réticulée 1 et la répartition des forces de pression sur une surface du crâne du foetus plus grande que ne le permettaient les appareils d'expulsion de l'Art antérieur, assure une meilleure préhension atraumatique. De plus, la faible épaisseur des tiges 1 et leurs extrêmités avantageusement spatulées, de même que leur facilité de glissement grâce à la disposition d'un revêtement approprié, permettent une introduction aisée dans l'espace autour de la tête foetale au niveau de l'excavation pelvienne.

L'appareil extracteur conforme à la présente invention présente en outre l'avantage, en raison de sa conception, de pouvoir être fabriqué à l'échelle industrielle à un prix de revient relativement faible, ce qui permet de concevoir la partie "applicateur" de l'appareil comme étant un instrument à usage unique, l'organe de traction étant, quant à lui, réutilisable.

Une telle conception permet d'utiliser un instrument stérilisé en fin de fabrication, livré sous emballage stérile, et d'éviter ainsi les opérations de stérilisation au niveau hospitalier qui sont relativement longues et requièrent une surveillance de la part du personnel hospitalier.

Ainsi que cela ressort de ce qui précède, l'invention ne se limite nullement à ceux de ses modes de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Appareil d'extraction de foetus par les voies génitales maternelles naturelles comprenant une carcasse constituée par une pluralité de tiges (1) flexibles disposées sensiblement en forme de corolle, solidaires à l'une de leurs extrémités d'une structure réticulée (4) en matériau souple, ladite structure s'étendant à l'intérieur de la corolle susdite, et une gaine tubulaire (2) creuse, caractérisé en ce que lesdites tiges sont solidaires à leurs extrémités opposées de la gaine tubulaire, et que ladite structure réticulée (4) se compose de deux réseaux imbriqués l'un dans l'autre, dont l'un est le réseau à mailles bloquées (12) et dont l'autre est un réseau à mailles libres (13), les tresses qui composent le réseau à mailles libres comportant à leur extrémité qui s'étend librement à l'intérieur de la corolle susdite, des brins de traction (5) fixés à ladite extrémité, et qui se terminent à leurs extrémités opposées en un noyau d'ancrage (6) apte à être reçu dans un organe de traction approprié.

2. Appareil selon la revendication 1, caractérisé en ce que les brins de traction (5) sont guidés dans la gaine tubulaire susdite (2).

3. Appareil selon l'une quelconque des revendications 1 et 2 ,caractérisé en ce que l'organe de traction comprend une cage (7) destinée à recevoir le noyau d'ancrage (6) susdit, sur une partie de laquelle est monté un manchon mobile (8) et dont l'extrémité opposée à celle sur laquelle est monté le manchon porte une poignée de traction (9).

4. Appareil selon la revendication 3, caractérisé en ce que le manchon (8) est monté mobile sur la partie d'extrémité de la cage d'ancrage (7) orientée vers la gaine tubulaire creuse (2) de l'appareil d'extraction.

5. Appareil selon la revendication 4 caractérisé en ce que ladite partie d'extrémité (10) est pourvue d'un filetage correspondant au filetage intérieur dont est également pourvu le manchon (8) susdit.

6. Appareil selon l'une quelconque des revendications 3 à 5, caractérisé en ce que le manchon (8) coopère avec une butée (11) solidaire de la gaine tubulaire (2) susdite.

## Claims

1. An instrument for extracting the fetus via the natural genital passages of the mother, the instrument comprising a hollow tubular sheath (2) and a body constituted by a plurality of flexible rods (1) disposed substantially in the form of a crown, each secured at one end to a reticulated structure (4) of flexible material, said structure extending inside the above-mentioned crown, the instrument being characterized in that each of said rods is secured at its opposite end to the tubular sheath, and in that said reticulated structure (4) comprises two networks that are interleaved within one another, one being a network of stopped loops (12) and the other being a network of free loops (13), the braids that make up the network of free loops having, at their ends that extend freely into the above-mentioned crown, respective traction strands (5) fixed thereto, the strands terminating at their opposite ends in an anchor knob (6) suitable for being received in an appropriate traction member.

2. An instrument according to claim 1, characterized in that the traction strands (5) are guided inside the above-mentioned tubular sheath (2).

3. An instrument according to claim 1 or 2, characterized in that the traction member comprises a cage (7) designed to receive the above-mentioned anchor knob (6), having a moving sleeve (8) mounted on a portion thereof, and having a traction handle (9) on its end opposite to the end on which the sleeve is mounted.

4. An instrument according to claim 3, characterized in that the sleeve (8) is moveably mounted on the end portion of the anchoring cage (7) directed towards the hollow tubular sheath (2) of the extraction instrument.

5. An instrument according to claim 4, characterized in that said end portion (10) is provided with a screw thread corresponding to the inside screw thread with which the above-mentioned sleeve (8) is also provided.

6. An instrument according to any one of claims 3 to 5, characterized in that the sleeve (8) co-operates with an abutment (11) secured to the above-mentioned tubular sheath (2).

## Patentansprüche

1. Vorrichtung zur Entnahme eines Fötus auf natürlichen genitalen Wegen der Mutter, mit einem Korpus, welcher aus einer Mehrzahl flexibler Stäbe (1) gebildet ist, die im wesentlichen in Form einer Krone angeordnet sind und mit einem ihrer Enden mit einem netzförmigen Gebilde (4) aus weichelastischem Material verbunden sind, wobei dieses Gebilde sich in das Innere der genannten Krone erstreckt, und die einem hohlen röhrenförmigen Mantel (2) aufweist, **dadurch gekennzeichnet, daß** die Stäbe an ihren dem röhrenförmigen Mantel gegenüberliegenden Enden verbunden sind und daß das netzförmige Gebilde (4) aus zwei ineinander übergreifenden Netzen gebildet ist, von denen eines ein Netz mit festen Maschen (12) und das andere ein Netz mit freien Maschen (13) ist, wobei das Geflecht, welches das Netz mit den freien Maschen bildet, an dem Ende, das sich frei in das Inneren der genannten Krone erstreckt, Zugfaden (5) aufweist, die an diesem Ende befestigt sind und an ihren gegenüberliegenden Enden in einen Verankerungskern (6) münden, welcher zur Aufnahme in einem geeigneten Zugelement vorgesehen ist.

2. Vorrichtung nach Anspruch 1, **dadurch gekennzeichnet, daß** die Zugfäden (5) in diesem röhrenförmigen Mantel (2) geführt sind.

3. Vorrichtung nach einem der Ansprüche 1 und 2, **dadurch gekennzeichnet, daß** das Zugelement ein Gehäuse (7) aufweist, welches zur Aufnahme des Verankerungskernes (6) dient, wobei an einem Teil desselben eine bewegliche Muffe (8) befestigt ist und dessen Ende, welches jenem an dem die Muffe befestigt ist, gegenüber liegt, eine Zughandhabe (9) aufweist.

4. Vorrichtung nach Anspruch 3, **dadurch gekennzeichnet, daß** die Muffe (8) beweglich an dem Endteil des Verankerungsgehäuses (7) befestigt ist, welcher gegen den hohlen röhrenförmigen Mantel (2) der Entnahmevorrichtung gerichtet ist.

5. Vorrichtung nach Anspruch 4, **dadurch gekennzeichnet, daß** dieser Endteil (10) mit einem Gewinde versehen ist, welches einem an der Muffe (8) vorgesehenen Innengewinde zugeordnet ist.

6. Vorrichtung nach einem der Ansprüche 3 bis 5, **dadurch gekennzeichnet, daß** die Muffe (8) mit einem Anschlag (11) zusammenwirkt, welcher mit dem röhrenförmigen Mantel verbunden ist.
